# EUROPÄISCHE PATENTSCHRIFT

(11) **EP 0 266 310 B1**
(45) Veröffentlichungstag und Bekanntmachung des Hinweises auf die Patenterteilung: **05.12.2001**
(21) Anmeldenummer: 87810609.5
(22) Anmeldetag: 22.10.1987
(51) Int. Cl.: C07D 491/04, B41M 5/145

(54) **Chromogene Phthalide**
Chromogenic phthalides
Phtalides chromogènes

(30) Priorität: 28.10.1986 CH 426886; 05.06.1987 CH 214887
(43) Veröffentlichungstag der Anmeldung: 04.05.1988
(73) Patentinhaber: Ciba Specialty Chemicals Holding Inc., 4057 Basel (CH)
(72) Erfinder: Phaff, Rox, Dr., CH-4310 Rheinfelden (CH); Bedekovic, Davor, Dr., CH-4105 Biel-Benken (CH)

(56) Entgegenhaltungen:
- EP-A- 0 181 646
- CH-A- 652 733
- CH-A- 653 353
- DE-A- 2 412 509
- DE-A- 2 412 640
- DE-A- 2 435 408
- DE-A- 2 435 640
- GB-A- 2 148 923
- US-A- 4 045 458
- CHEMICAL ABSTRACTS, Band 104, Nr. 14, 7. April 1986, COLUMBUS, OHIO, USA, KAWAI, HAJIME; NAKAI, ZOSHUHIKO ( YAMADA CHEMICAL CO., LTD ) " IMAGE RECORDING SYSTEMS " Seite 682, Spalte 1, Zusammenfassung-Nr. 120 088x

## Beschreibung

Die vorliegende Erfindung betrifft chromogene Phthalide, Verfahren zu ihrer Herstellung und ihre Verwendung als Farbbildner in druckempfindlichen oder wärmeempfindlichen Aufzeichnungsmaterialien.

Aus den Druckschriften CH-A-653353, DE-A-2423534, EP-A-206114, US-A-3829322, GB-A-2148923 und US-A-4045458 sind Indol-3-yl-phthalide bzw. Indol-3-ylazaphthalide bekannt, in denen der Indolylrest in 3-Stellung mit dem Phthalidgerüst verbunden ist. Desweiteren werden in den Druckschriften DE-A-2435408, DE-A-2412509, DE-A-2412640 und DE-A-2435640 Pyridincarbonsäure-Lactone beschrieben, die einen Thianaphthenylrest aufweisen können. Aus der DE-A-3416767 sind ferner 3,3-Bis-Indol-3'-ylazaphthalide bekannt.

Demgegenüber entsprechen erfindungsgemässe Verbindungen der allgemeinen Formel 1 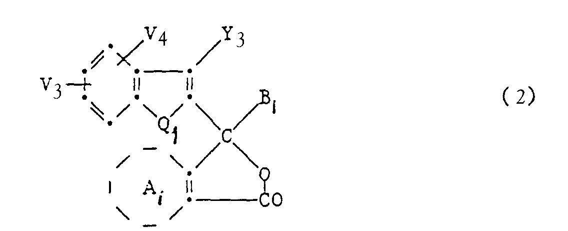 worin V₃ für Niederalkoxy oder -NR₅R₆ und V₄ für Wasserstoff, Halogen oder Niederalkyl steht, A₁ einen gegebenenfalls durch Halogen oder Diniederalkylamino substituierten Benzolring,
B₁ einen substituierten Phenylrest der Formel 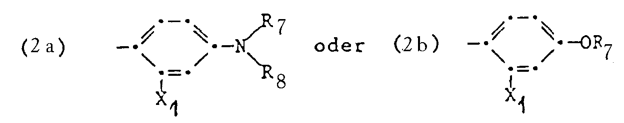 oder einen 3-Indolylrest der Formel 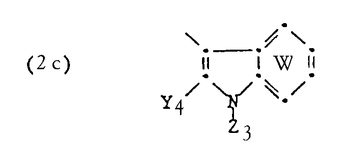 Q₁ 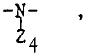 R₅, R₆, R₇ R₈, unabhängig voneinander, je Alkyl mit höchstens 12 Kohlenstoffatomen oder Cycloalkyl mit 5 oder 6 Kohlenstoffatomen, Benzyl, Phenethyl oder Phenyl, oder die Substituentenpaare (R₅ und R₆) und (R₇ und R₈), unabhängig voneinander, je zusammen mit dem sie verbindenden Stickstoffatom einen Pyrrolidino, Piperidino oder Morpholino Rest,
Y₃ Niederalkyl,
Y₄ Wasserstoff, Phenyl oder Methyl,
Z₃ und Z₄, unabhängig voneinander, je Wasserstoff, C₁-C₈-Alkyl, Acetyl, Propionyl oder Benzyl,
X₁ Wasserstoff, Halogen, Niederalkyl, C₁-C₈-Alkoxy, Benzyloxy oder die Gruppe -NT₃T₄,
T₃ und T₄, unabhängig voneinander, je Wasserstoff, Niederalkyl, Niederalkylcarbonyl oder Benzoyl und
W Wasserstoff oder Halogen bedeutet.

Niederalkyl und Niederalkoxy stellen bei der Definition der Reste der Phthalide solche Gruppen oder Gruppenbestandteile dar, die 1 bis 5, insbesondere 1 bis 3 Kohlenstoffatome aufweisen. Beispiele für derartige Gruppen sind Ethyl, n-Propyl, Isopropyl, n-Butyl, sek.-Butyl, Amyl oder Isoamyl bzw. Methoxy, Ethoxy, Isopropoxy, Isobutoxy oder tert.Butoxy.

Halogen bedeutet beispielsweise Fluor, Brom oder vorzugsweise Chlor.

Als C₁-C₈-Alkoxyrest kann X₁ eine geradkettige oder verzweigte Gruppe sein, wie z.B. Methoxy, Ethoxy, Isopropoxy, tert.-Butoxy, n-Hexyloxy oder Octyloxy.

Bevorzugt sind auch Verbindungen der Formel (2), in der Q₁ 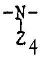 und B₁ einen substituierten Phenylrest der Formel (2a) oder (2b) bedeuten.

Stellen die Substituenten R₅, R₆, R₇, R₈, Z₃ und Z₄ Alkylgruppen dar, so können sie geradkettige oder verzweigte Alkylreste sein. Beispiele solcher Alkylreste sind Methyl, Ethyl, n-Propyl, Isopropyl, n-Butyl, sek.-Butyl, tert.-Butyl, Amyl, Isoamyl, n-Hexyl, 2-Ethyl-hexyl, n-Heptyl, n-Octyl, Isooctyl, n-Nonyl, Isononyl oder n-Dodecyl.

Beispiele für Cycloalkyl in der Bedeutung der R- Reste sind Cyclopentyl, oder vorzugsweise Cyclohexyl. Die Cycloalkylreste können einen oder mehrere C₁-C₄-Alkylreste, vorzugsweise Methylgruppen, enthalten und weisen insgesamt 5 bis 10 Kohlenstoffatome auf.

Die Substituenten R₅, R₆, R₇ und R₈ sind vorzugsweise Cyclohexyl, oder in erster Linie Niederalkyl, wie z.B. Methyl oder vor allem Ethyl. -NR₅R₆ und -NR₇R₈ sind bevorzugt auch Pyrrolidinyl.

X₁ kann vorteilhafterweise Wasserstoff, Halogen, Niederalkyl, wie z.B. Methyl, Benzyloxy, C₁-C₈-Alkoxy, in erster Linie Niederalkoxy, wie z.B. Methoxy, Ethoxy, Isopropoxy oder tert.Butoxy, oder die Gruppe -NT₃T₄ sein, wobei von den Resten T₃ und T₄ eines vorzugsweise Niederalkylcarbonyl oder Niederalkyl und das andere Wasserstoff oder Niederalkyl ist. Der Acylrest ist Niederalkylcarbonyl, wie z.B. Acetyl oder Propionyl. Vorzugsweise ist X₁ Acetylamino, Dimethylamino, Benzyloxy oder besonders Niederalkoxy und vor allem Ethoxy oder Wasserstoff.

Die N-Substituenten Z₃ und Z₄ sind vorzugsweise Benzyl, Acetyl, Propionyl oder besonders Alkyl mit 1 bis 8 Kohlenstoffatomen, wie z.B. Methyl, Ethyl, n-Butyl oder vor allem n-Octyl.

Y₃ ist vorzugsweise Niederalkyl, wie z.B. Methyl, Ethyl, Isopropyl, während Y₄ vorzugsweise Phenyl oder vor allem Methyl ist.

V₃ ist vorzugsweise -NR₅R₆ oder vor allem Niederalkoxy, während V₂ vorzugsweise Wasserstoff, Halogen oder Methyl ist. V₃ befindet sich vorzugsweise in 4-Stellung oder besonders in 6-Stellung. Als Substituent befindet sich V₄ vorzugsweise in p-Stellung zu Q₁(5-Stellung).

Der Ring A₁ ist vorzugsweise ein 1,2-Benzorest, der unsubstituiert oder durch Diniederalkylamino, wie z.B. Dimethylamino, oder vier Chloratome oder Bromatome substituiert ist.

Von besonderem Interesse.sind Phthalide der Formel 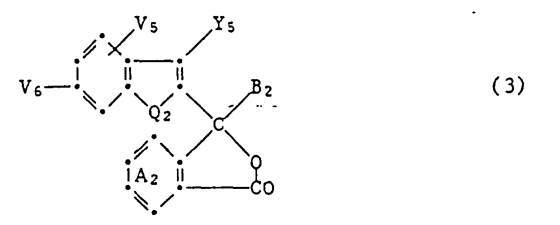 worin
der Benzolring A₂ unsubstituiert oder durch Niederalkylamino oder Halogen substituiert ist,

B₂ einen substituierten Phenylrest der Formel 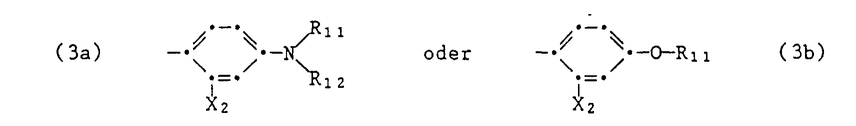 oder einen 3-Indolylrest der Formel 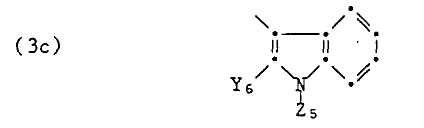 Q₂ 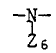 V₅ Wasserstoff oder Halogen,
V₆ -NR₉R₁₀ oder Niederalkoxy,
R₉, R₁₀, R₁₁ und R₁₂, unabhängig voneinander, je Niederalkyl, Cyclohexyl oder Benzyl oder die Substituentengruppen -NR₉R₁₀ und -NR₁₁R₁₂ Pyrrolidino, Piperidino oder Morpholino,
X₂ Wasserstoff, Methyl, Niederalkoxy, Benzyloxy, Acetylamino, Propionylamino, Benzoylamino oder Diniederalkylamino,
Y₅ Niederalkyl, wie z.B. Methyl,
Y₆ Phenyl oder vor allem Methyl, und
Z₅ und Z₆, unabhängig voneinander, je Wasserstoff, Alkyl mit 1 bis 8 Kohlenstoffatomen oder Benzyl bedeuten.

Bevorzugt sind auch Verbindungen der Formel (3), in der A₂ einen unsubstituierten oder durch Dimethylamino substituierten 1,2-Benzorest,
B₂ einen substituierten Phenylrest der Formel (3a) oder (3b),
Q₂ 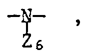 V₅ Wasserstoff oder Chlor,
V₆ -NR₉R₁₀,
R₉ Methyl, Ethyl oder Cyclohexyl,
R₁₀ Methyl oder Ethyl oder NR₉R₁₀ Pyrrolidinyl,
R₁₁ und R₁₂ je Methyl oder Ethyl oder -NR₁₁R₁₂ Pyrrolidinyl,
Y₅ Methyl oder Ethyl,
X₂ Wasserstoff, Methoxy oder Ethoxy und
Z₆ C₁-C₈-Alkyl, besonders Methyl oder Ethyl bedeuten.

Ganz besonders bevorzugt sind Verbindungen der Formel (3), in der A₂ einen unsubstituierten oder durch Dimethylamino substituierten 1,2-Benzorest,
B₂ einen substituierten Phenylrest der Formel (3a),
Q₂ 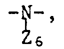 V₅ Wasserstoff oder Chlor,
V₆ Niederalkoxy, besonders Methoxy,
R₁₁ und R₁₂ je Methyl, Ethyl oder -NR₁₁R₁₂ Pyrrolidinyl,
Y₅ Methyl oder Ethyl,
X₂ Wasserstoff, Methoxy oder Ethoxy und
Z₆ C₁-C₈-Alkyl, besonders Methyl, Ethyl oder n-Octyl bedeuten.

Die erfindungsgemässen Phthalide der Formeln (2) und (3) stellen neue chromogene Verbindungen dar und können nach an sich bekannten Methoden hergestellt werden.

Ein Verfahren zur Herstellung der Verbindungen der Formel (2) besteht darin, dass man in beliebiger Reihenfolge ein Mol eines Anhydrids der Formel 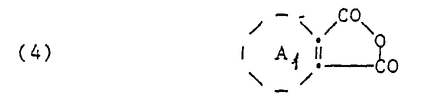 mit einem Mol einer Verbindung der Formel 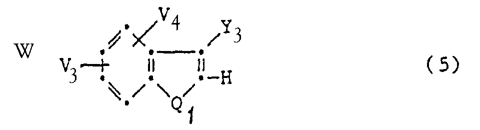 und einem Mol einer Verbindung der Formel

B₁-H (6)

umsetzt, worin in den Formeln (4), (5) und (6) A₁, B₁, Q₁, V₃, V₄, und Y₃ die angegebene Bedeutung haben.

Vorteilhafterweise werden die erfindungsgemässen Laktone dadurch hergestellt, dass man eine Verbindung der Formel (5) mit einer Verbindung der Formel 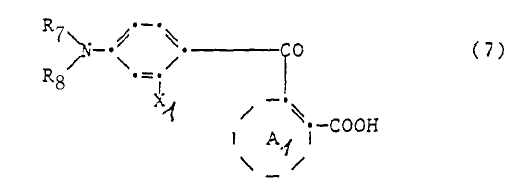 oder der Formel 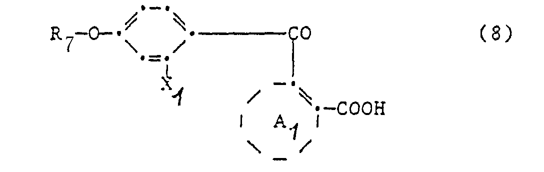 oder der Formel 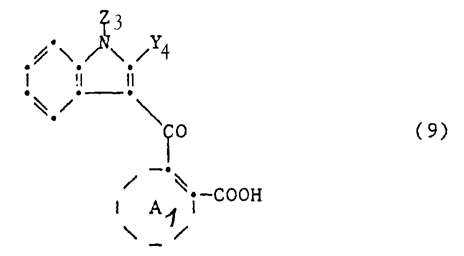 umsetzt, worin in den Formeln (7), (8) und (9) A₁, R₇, R₈, X₁, Z₃ und Y₄ die angegebene Bedeutung haben.

Die Umsetzungen werden vorzugsweise so durchgeführt, dass man die Reaktionskomponenten in Anwesenheit eines sauren Entwässerungsmittels bei einer Temperatur von 20°C bis 140°C zur Reaktion bringt. Beispiele für derartige Kondensationsmittel sind Essigsäureanhydrid, Zinkchlorid, Schwefelsäure, Phosphorsäure und Phosphoroxychlorid.

Die Isolierung des Endproduktes der Formel (2) erfolgt in allgemein bekannter Weise durch Einstellen des Reaktionsgemisches auf einen pH-Wert von mindestens 6, vorzugsweise 7 bis 11, z.B. mit Alkalien, wie z.B. Alkalimetallhydroxiden, Ammoniak, Alkalimetallcarbonaten oder -bicarbonaten und Abtrennen des gebildeten Niederschlags, Waschen und Trocknen oder durch Behandeln mit geeigneten organischen Lösungsmitteln, wie z.B. Methanol, Isopropanol, Benzol, Chlorbenzol oder vor allem Toluol oder Toluol/Methanol.

Die Ausgangsprodukte der Formel (5) sind neu oder sind z.B. in der EP-A-106800 beschrieben. Allgemein gesagt können sie z.B. durch Umsetzung einer 3-Aminobenzolverbindung der Formel 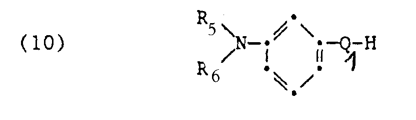 mit einer Verbindung der Formel (11) Y₃-CO-CH₂-Hal, worin Hal für Halogen steht bzw. einem entsprechenden Acetal davon z.B. Chloraceton oder 2-Chlor-1,1-diethoxy-ethan, in Gegenwart einer Base z.B. Kaliumcarbonat und gegebenenfalls in einem organischen Lösungsmittel z.B. Dimethylformamid erhalten. In Formeln (10) und (11) haben R₅, R₆, Q₁ und Y₃ die oben angegebene Bedeutung.

Die Ausgangsstoffe der Formeln (4), (6), (7) und (9) sind z.B. in den deutschen Offenlegungsschriften DE-A-2 265 233, DE-A-2 514 934, DE-A-3 247 059, DE-A-3 319 978 und DE-A-3 600 725 und in den europäischen Patentanmeldungen EP-A-82 822 und EP-A 140 839 beschrieben.

Die Phthalide der Formeln (2) bis (3) sind normalerweise farblos oder höchstens schwach gefärbt. Wenn diese Farbbildner mit einem vorzugsweise sauren Entwickler, d.h. einem Elektronenakzeptor, in Kontakt gebracht werden, so ergeben sie je nach Bedeutung von A₁, B₁ und Q₁ und dem verwendeten Entwickler intensive grüne, grün-blaue, blaue oder violett-blaue Farbtöne, die sublimations- und lichtecht sind. Grün entwickelnde Farbbildner zeigen zudem Absorption bis in den IR-Bereich. Das entsprechende Schrittbild kann somit maschinell z.B. mit Laser abgelesen werden. Die Phthalide der Formeln (2) bis (3) sind auch sehr wertvoll im Gemisch mit einem oder mehreren anderen bekannten Farbbildner, z.B. 3,3-(Bis-aminophenyl-)-phthaliden, (3,3-Bis-indolyl-)-phthaliden, 3-Aminc-fluoranen, 2,6-Diaminofluoranen, 2,6-Di-amino-3-methyl-fluoranen, Leukoauraminen, Spiropyranen, Spirodipyranen, Chromenopyrazolen, Chromenoindolen, Phenoxazinen, Phenothiazinen, Chinazolinen, Rhodaminlaktamen, Carbazolylmethanen oder weiteren Triarylmethan-leukofarbstoffen, um blaue, marine-blaue, graue oder schwarze Färbungen zu ergeben.

Die Phthalide der Formeln (2) bis (3) zeigen sowohl auf aktivierten Tonen, wie auch auf phenolischen Unterlagen eine ausgezeichnete Farbintensität und Lichtechtheit. Sie eignen sich vor allem als sich schnell entwickelnde Farbbildner für die Verwendung in einem wärmeempfindlichen oder insbesondere druckempfindlichen Aufzeichnungsmaterial, das sowohl Kopier- als auch Registriermaterial sein kann. Sie zeichnen sich dadurch aus, dass sie pH stabil und in den Kapselölen hervorragend löslich sind. Nach Belichtung in CB-Blatt weisen sie eine geringe Abnahme der Farbstärke (CB-Desaktivierung) auf.

Ein druckempfindliches Material besteht beispielsweise aus mindestens einem Paar von Blättern, die mindestens einen Farbbildner der Formeln (2) bis (3) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxyd, Aluminiumoxid, Aluminiumsulfat, Aluminiumphosphat, Zinkchlorid, Zinknitrat, aktiviertes Kaolin oder irgendein beliebiger Ton. Als Entwickler können auch sauer reagierende, organische Verbindungen, wie z.B. gegebenenfalls ringsbustituierte Phenole, Resorcine, Salicylsäuren, wie z.B. 3,5-Bis-(α,α-di-methylbenzyl-)-salicylsäure oder 3,5-Bis-(α-methylbenzyl)-salicylsäure oder Salicylsäureester und deren Metallsalze, z.B. Zinksalze, sowie ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate oder die Kondensationsprodukte von p-substituierten Phenolen mit Formaldehyd. Die letzteren können auch mit Zink modifiziert sein.

Die Entwickler können zusätzlich auch im Gemisch mit an sich unreaktiven oder wenig reaktiven Pigmenten oder weiteren Hilfstoffen wie Kieselgel oder UV-Absorbern, wie z.B. 2-(2'-Hydroxyphenyl-)-benztriazolen eingesetzt werden. Beispiele für solche Pigmente sind: Talk, Titandioxid, Aluminiumoxid, Aluminiumhydroxyd, Zinkoxid, Kreide, Tone wie Kaolin, sowie organische Pigmente, z.B. Harnstoff-Formaldehydkondensate (BET-Oberfläche 2-75 m²/g) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. polyhalogeniertes Paraffin oder Diphenyl, wie Chlorparaffin, Monochlordiphenyl oder Trichlordiphenyl, ferner Tricresylphosphat, Di-n-butylphthalat, Dioctylphthalat, Trichlorbenzol, Trichlorethylphosphat, aromatische Ether, wie Benzylphenylether, Kohlenwasserstofföle, wie Paraffin oder Kerosin, z.B. mit Isopropyl, Isobutyl, sek-Butyl oder tert.-Butyl alkylierte Derivate von Diphenyl, Naphthalin oder Terphenyl, Dibenzyltoluol, partiell hydriertes Terphenyl, mono- bis tetra-C₁-C₃-alkylierte Diphenylalkane, Dodecylbenzol, benzylierte Xylole, oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen. Bei der Einkapselung zeichnen sich die erfindungsgemässen Phthalide dadurch aus, dass sie ausserordentlich hohe pH-Beständigkeit z.B. in einem pH-Bereich von 4 bis 10 zeigen.

Die Kapselwände können durch Koazervationskräfte gleichmässig um die Tröpfchen der Farbbildnerlösung herum gebildet werden, wobei das Einkapselungsmaterial z.B. in der US-Patentschrift 2,800,457 beschrieben ist. Die Kapseln können vorzugsweise auch aus einem Aminoplast oder modifizierten Aminoplasten durch Polykondensation gebildet werden, wie es in den britischen Patentschriften 989,264, 1,156,725, 1,301,052 und 1,355,124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formeln (2) bis (3) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Uebertragungsblattes und der Elektronenakzeptor in Form einer Schicht auf der Vorderseite eines Empfangsblattes vorhanden sind.

Eine andere Anordnung der Bestandteile besteht darin, dass die den Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Verbindungen der Formeln (2) bis (3) können auch als Farbbildner in einem thermoreaktiven Aufzeichnungsmaterial verwendet werden. Dieses enthält in der Regel mindestens einen Schichtträger, einen Farbbildner, und einen Elektronenakzeptor und gegebenenfalls auch ein Bindemittel und/oder Wachs.

Thermoreaktive Aufzeichnungssysteme umfassen, z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-PS 12,51,348 beschrieben sind, z.B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäure-methylester oder -benzylester, 4-Hydroxydiphenylsulfon, 4'-Hydroxy-4-methyldiphenylsulfon, 4'-Hydroxy-4-isopropoxydiphenylsulfon, 4-Hydroxyacetophenon, 2,2'-Dihydroxydiphenyl, 4,4'-Cyclohexylidendiphenol, 4,4'-Isopropylidendiphenol, 4,4'-Isopropyliden-bis-(2-methylphenol), ein Antipyrinkomplex von Zinkthiocyanat, ein Pyridinkomplex von Zinkthiocyanat, 4,4-Bis-(4-hydroxyphenyl)valeriansäure, Hydrochinon, Pyrogallol, Phloroglucin, p-, m-, o-Hydroxybenzoesäure, Gallussäure, 1-Hydroxy-2-naphthoesäure sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Phthalide und der Entwickler in Wasser schwer löslich oder unlöslich sind. Das Bindemittel sollte in der Lage sein, den Farbbildner und den Entwickler bei Raumtemperatur zu dispergieren und zu fixieren. Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine, Stärke oder veretherte Maisstärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumhydroxyd, Calciumcarbonat (z.B. Kreide), Tone oder auch organische Pigmente, wie z.B. Harnstoff-Formaldehydpolymerisate enthalten. Um zu bewirken, dass nur innnerhalb eines begrenzten Temperaturbereiches die Farbe gebildet wird, können Substanzen, wie Harnstoff, Thioharnstoff, Diphenylthioharnstoff, Acetamid, Acetanilid, Benzolsulfanilid, Stearinsäureamid, Phthalsäureanhydrid, Metallstearate, wie z.B. Zinkstearat, Phthalsäurenitril, Dimethylterephthalat oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanawachs, Paraffinwachs, Polyethylenwachs, Kondensate höherer Fettsäureamide und Formaldehyde und Kondensate höherer Fettsäuren und Ethylendiamin.

Eine weitere Anwendung der Verbindungen der Formeln (2) bis (3) ist die Herstellung eines Farbbildes mittels photohärtbarer Mikrokapseln, wie sie z.B. in der DE-OS 3 247 488 beschrieben sind.

In den folgenden Beispielen beziehen sich die angegebenen Prozentsätze, wenn nichts anderes angegeben ist, auf das Gewicht.

### Beispiel 1:

3,12 g 4,4'-Bis-dimethylaminobenzophenon-2-carbonsäure und 1,9 g 1-Ethyl-3-methyl-6-methoxyindol werden in 30 ml Essigsäureanhydrid 3 Stunden bei 40°C verrührt. Das Reaktionsgemisch wird auf Eis gegossen, mit NaOH basisch gestellt und mit Toluol extrahiert. Die organische Phase wird abgebrennt, mit Aktivkohle behandelt, getrocknet und eingedampft. Der Rückstand wird einmal aus Diethylether und einmal aus Toluol umkristallisiert. Man erhält 3,35 g 3-(4-Dimethylaminophenyl)-3-(1-ethyl-3-methyl-6-methoxy-indol-2-yl)-6-dimethylaminophthalid der Formel

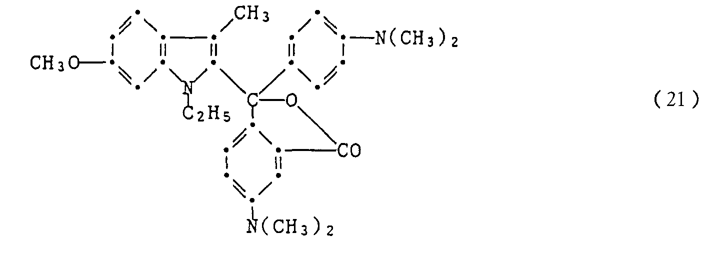

Schmelzpunkt 224-225°C. Auf saurem Ton ergibt diese Verbindung einen blauen Farbton.

Das verwendete 1-Ethyl-3-methyl-6-methoxyindol wird wie folgt hergestellt:
4,6 g 3-Methyl-6-Methoxyindol [Chem. Ber. 98, 1727 (1965)] werden in
29 ml Dimethylsulfoxid vorgelegt. Dann werden 6,6 ml 10n KOH_{aq} zugegeben und während 30' bei 20°C 3,25 g Ethylbromid zugetropft. Nach 1 Stunde wird das Reaktionsgemisch auf Wasser gegossen und mit Toluol extrahiert. Die Chromatographie an Kieselgel (Hexan-Ether 5:1) ergibt 3,9 g 1-Ethyl-3-methyl-6-methoxyindol als Oel.

### Beispiel 2:

3 g 4-Dimethylamino-4'-methoxybenzophenon-2-carbonsäure und 1,9 g 1-Ethyl-3-methyl-6-methoxyindol werden in 30 ml Essigsäureanhydrid 4 Stunden bei 40°C gerührt. Dann wird auf Eis ausgetragen, mit Natriumhydroxidlösung basisch gestellt und mit Toluol extrahiert. Die Toluolphase wird mit Aktivkohle behandelt, über Magnesiumsulfat getrocknet und eingedampft. Man erhält 4,45 g 3-(4-Methoxyphenyl)-3-(1-ethyl-3-methyl-6-methoxyindol(-2-yl)-6-dimethylaminophthalid der Formel

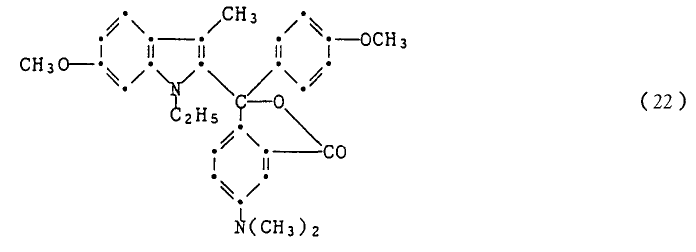

Schmelzpunkt 80-85°C. Auf saurem Ton ergibt die Verbindung eine grünblaue Farbe.

### Beispiel 3:

3,78 g (2-Methyl-1-n-octylindol-3-yl)-(2-carboxyphenyl)-keton und 1,81 g 1-Ethyl-3-methyl-6-methoxyindol werden in 30 ml Essigsäureanhydrid 8 Stunden bei 40°C gerührt. Das Reaktionsgemisch wird auf Eis gegossen, mit konz. Natriumhydroxidlösung basisch gestellt und mit Toluol extrahiert. Die Toluolphase wird einmal mit Wasser gewaschen, abgetrennt, mit Aktivkohle behandelt, über Natriumsulfat getrocknet und eingedampft. Nach Umkristallisieren aus einer Mischung von Hexan und Diethylether (3:2) erhält man 2,2 g einer Verbindung der Formel 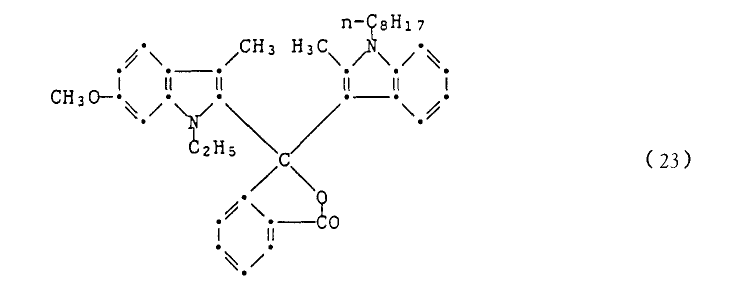 Schmelzpunkt 95-140°C. Auf saurem Ton ergibt diese Verbindung einen blauen Farbton.

### Beispiel 4:

2,58 g (4-Dimethylaminophenyl)-(2-carboxyphenyl)-keton und 1,81 g 1-Ethyl-3-methyl-6-methoxyindol werden in 30 ml Essigsäureanhydrid 4 Stunden bei 40°C gerührt, Das Reaktionsgemisch wird auf Eis gegossen, mit konz. Natriumhydroxidlösung basisch gestellt und mit Toluol extrahiert. Die organische Phase wird mit Wasser gewaschen, mit Aktivkohle behandelt, über Natriumsulfat getrocknet und eingedampft. Der Rückstand wird in Dimethylether verrührt, abfiltriert und getrocknet. Man erhält 2,85 g einer Verbindung der Formel

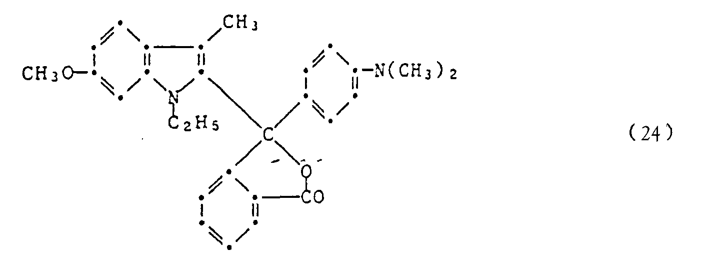

Schmelzpunkt 177-178°C. Auf saurem Ton ergibt diese Verbindung eine türkische Färbung.

Auf gleiche Art und Weise wie in Beispiel 1 beschrieben, können unter Verwendung der entsprechenden Ausgangsstoffe auch die in der Tabelle 1 aufgeführten Farbbildner der Formel (25) hergestellt werden.

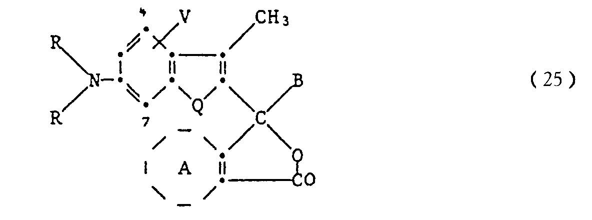

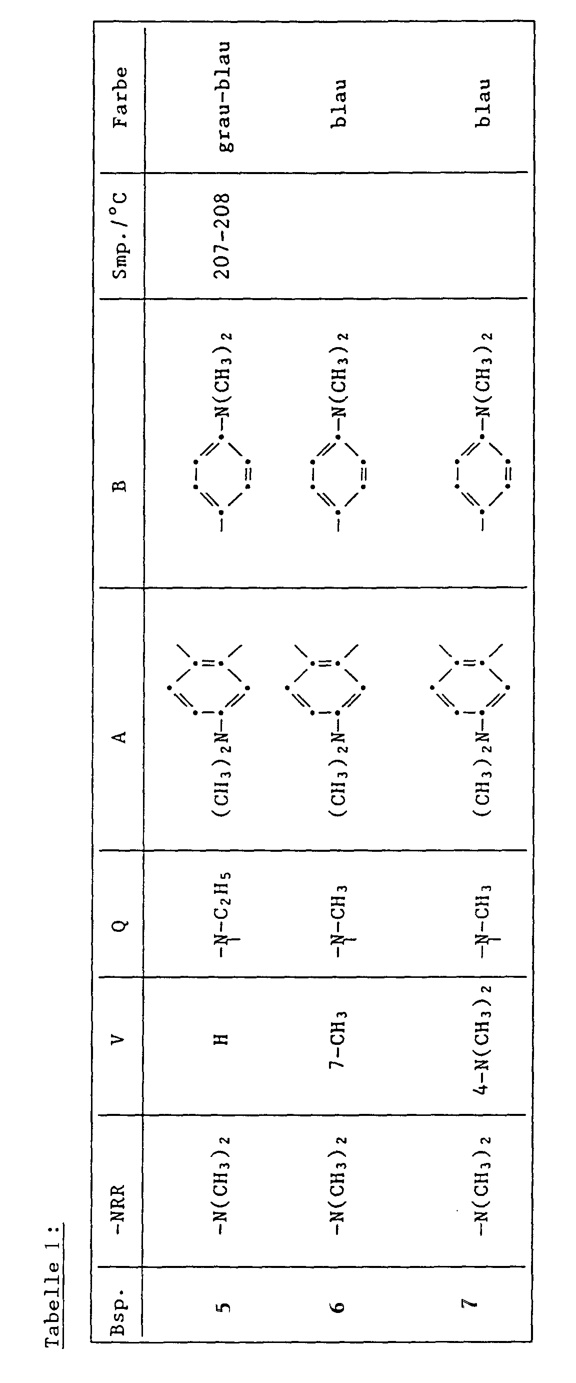

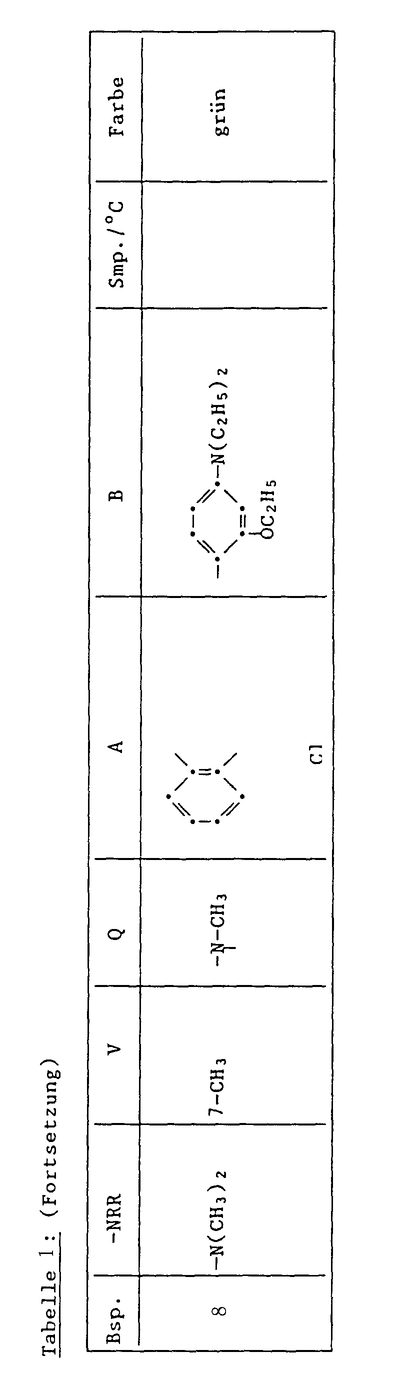

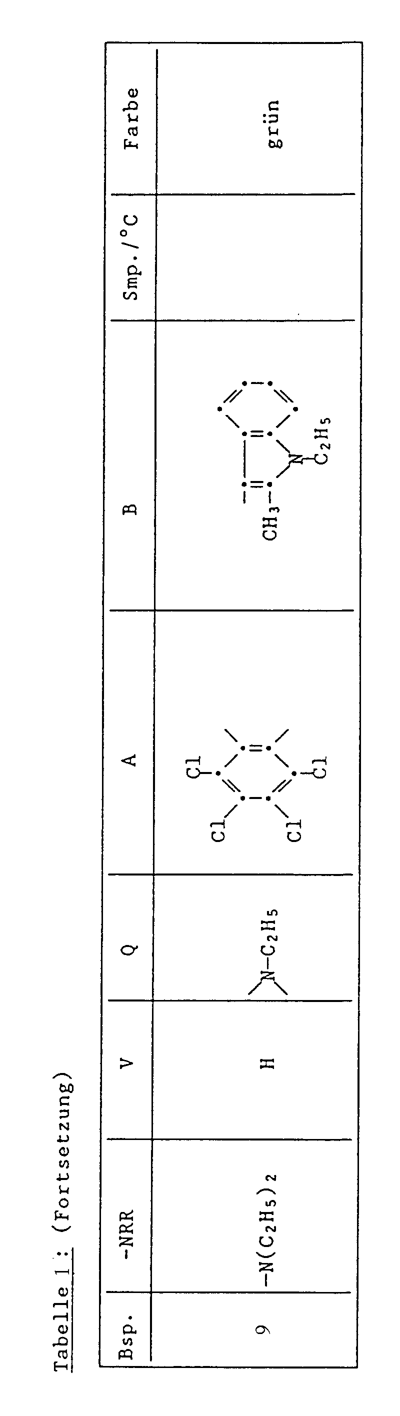

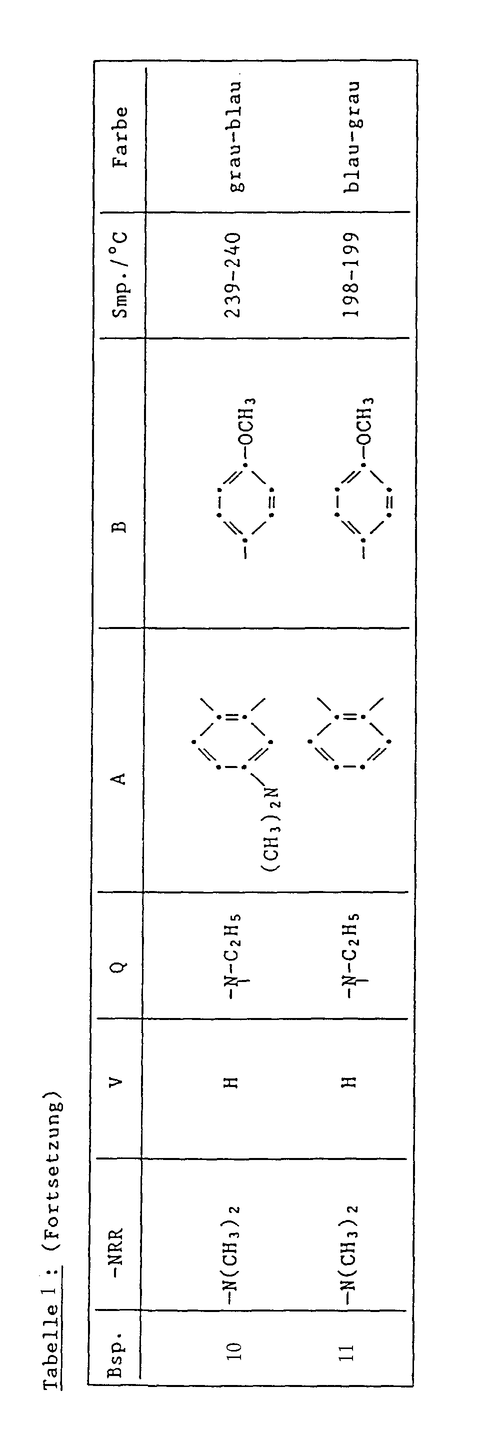

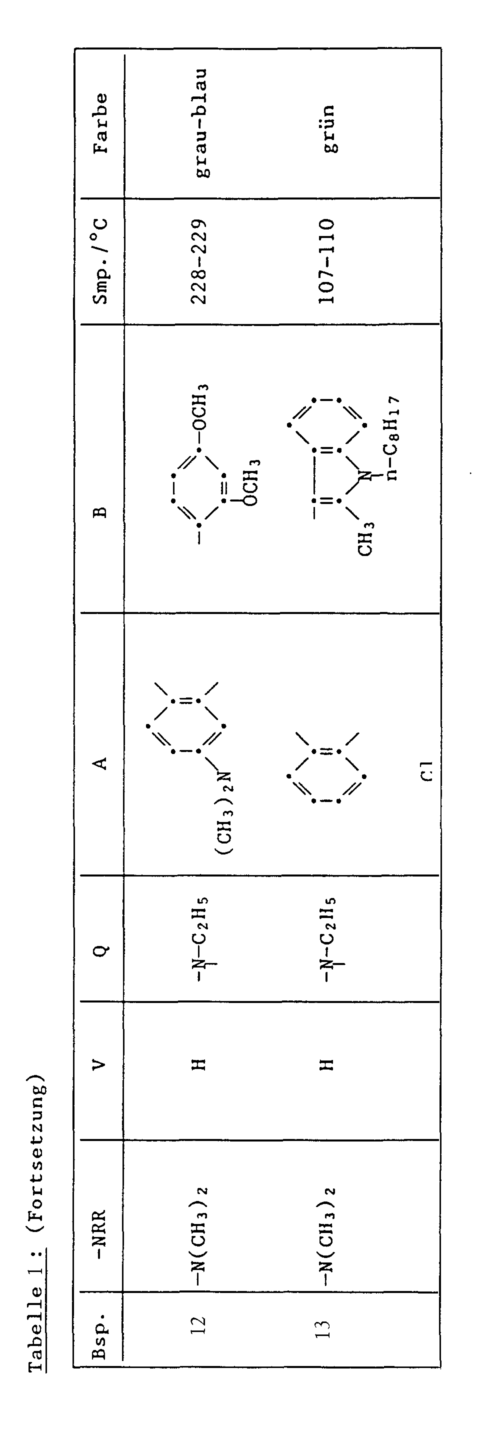

### Beispiel 14: Herstellung eines druckempfindlichen Kopierpapiers

Eine Lösung von 3 g des Phthalides der Formel (21) (Beispiel 1) in 80 g Diisopropylnaphthalin und 17 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit Aktivton als Farbentwickler beschichtet. Das erste den Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive blaue Kopie, die ausgezeichnet lichtecht ist.

Eine entsprechende intensive, lichtechte blaue bzw. grüne Kopie wird auch bei Verwendung jedes der anderen in den Herstellungs-Beispielen angegebenen Farbbildner der Beispiele 2 bis 13 erzielt.

Beispiel 15: 1 g des Phthalides gemäss Beispiel 6 wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1:1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 g/m² mit einer Mischung bestehend aus 1 Teil eines Amidwachses, 1 Teil eines Stearinwachses und 1 Teil Zinkchlorid beschichtet ist. Durch Schreiben mit der Hand oder mit der Schreibmaschine auf dem oberen Blatt wird Druck ausgeübt, und es entwickelt sich sofort auf dem mit dem Farbbildner beschichteten Blatt eine intensive und lichtechte blaue Farbe.

### Beispiel 16: Herstellung eines wärmeempfindlichen Aufzeichnungsmaterials

In einer Kugelmühle werden 32 g 4,4'Isopropylidendiphenol (Bisphenol A), 3,8 g Distearylamid des Ethylendiamins, 39 g Kaolin, 20 g eines zu 88 % hydrolysierten Polyvinylalkohols und 500 ml Wasser gemahlen bis die Teilchengrösse ca. 5 µm beträgt. In einer zweiten Kugelmühle werden 6 g des Phthalides gemäss Beispiel 1, 3 g eines zu 83 % hydrolysierten Polyvinylalkohols und 60 ml Wasser zu einer Teilchengrösse von ca. 3 µm gemahlen.

Die beiden Dispersionen werden zusammengegeben und mit einem Trockenauftragsgewicht von 5,5 g/m² auf ein Papier gestrichen. Durch Berührung des Papiers mit einem erhitzten Kugelschreiber wird eine intensive blaue Farbe erhalten, die eine ausgezeichnete Licht- und Sublimierechtheit hat.

Intensive und lichtechte blaue bzw. grüne Farbe kann auch bei Verwendung jedes der anderen Farbbildner gemäss Beispielen 2 bis 13 erhalten werden.

## Patentansprüche

1. Chromogene Phthalide der Formel 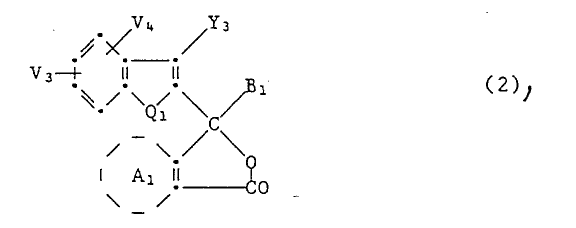 worin
V₃ Niederalkoxy oder -NR₅R₆,
V₄ Wasserstoff, Halogen oder Niederalkyl,
A₁ einen gegebenenfalls durch Halogen oder Diniederalkylamino substituierten 1,2- Benzorest.
B₁ einen substituierten Phenylrest der Formel 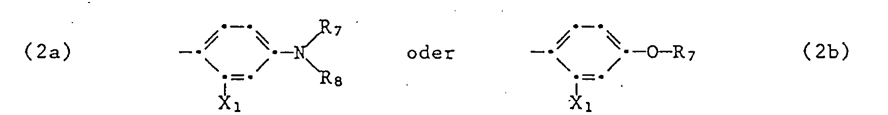 oder einen 3-Indolylrest der Formel 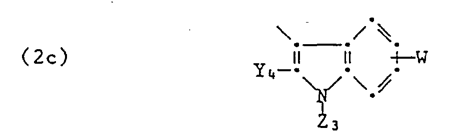 Q₁ 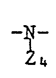 R₅, R₆, R₇ und R₈, unabhängig voneinander, je Alkyl mit höchstens 12 Kohlenstoffatomen, C₅-C₆-Cycloalkyl, Benzyl, Phenethyl oder Phenyl, oder die Substituentenpaare (R₅ und R₆) und (R₇ und R₈), unabhängig voneinander, je zusammen mit dem sie verbindenden Stickstoffatom Pyrrolidino, Piperidino oder Morpholino,
Y₃ Niederalkyl,
Y₄ Wasserstoff, Methyl oder Phenyl,
Z₃ und Z₄, unabhängig voneinander, je Wasserstoff, C₁-C₈-Alkyl, Acetyl, Propionyl oder Benzyl,
X₁ Wasserstoff, Halogen, Niederalkyl, C₁-C₈-Alkoxy, Benzyloxy oder die Gruppe -NT₃T₄,
T₃ und T₄, unabhängig voneinander, je Wasserstoff, Niederalkyl, Niederalkylcarbonyl oder Benzoyl und
W Wasserstoff oder Halogen bedeuten.

2. Phthalide gemäss Ansprüch 1, **dadurch gekennzeichnet, dass** in Formel (2) B₁ ein substituierter Phenylrest der Formel (2a) oder (2b) ist.

3. Phthalide gemäss Anspruch 1, **dadurch gekennzeichnet, dass** sie der Formel 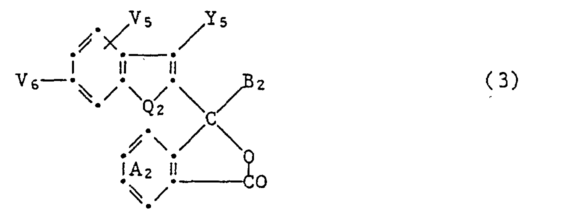 entsprechen, worin der Benzolring A₂ unsubstituiert oder durch Niederalkylamino oder Halogen substituiert ist,
B₂ einen substituierten Phenylrest der Formel 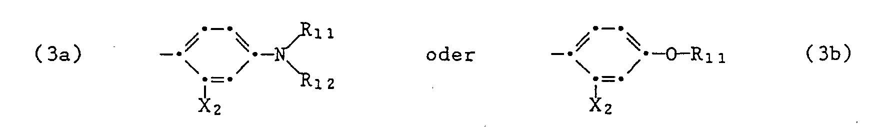 oder einen 3-Indolylrest der Formel 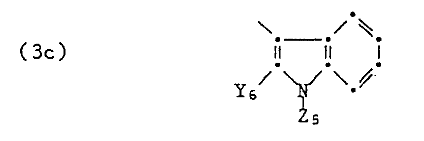 Q₂ 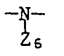 V₅ Wasserstoff oder Halogen,
V₆ -NR₉R₁₀ oder Niederalkoxy,
R₉, R₁₀, R₁₁ und R₁₂, unabhängig voneinander, je Niederalkyl, Cyclohexyl oder Benzyl oder die Substituentengruppen -NR₉R₁₀ und -NR₁₁R₁₂ Pyrrolidino, Piperidino oder Morpholino,
X₂ Wasserstoff, Methyl, Niederalkoxy, Benzyloxy, Acetylamino, Propionylamino, Benzoylamino oder Diniederalkylamino,
Y₅ Niederalkyl,
Y₆ Methyl oder Phenyl, und
Z₅ und Z₆, unabhängig voneinander, je Wasserstoff, Alkyl mit 1 bis 8 Kohlenstoffatomen oder Benzyl bedeuten.

4. Phthalide gemäss Anspruch 3, **dadurch gekennzeichnet, dass** in Formel (3)
A₂ einen unsubstituierten oder durch Dimethylamino substituierten 1,2-Benzorest,
B₂ einen substituierten Phenylrest der Formel (3a) oder (3b), Q₂ 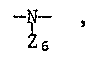 V₅ Wasserstoff oder Chlor,
V₆ -NR₉R₁₀,
R₉ Methyl, Ethyl oder Cyclohexyl,
R₁₀ Methyl oder Ethyl oder -NR₉R₁₀ Pyrrolidinyl,
R₁₁ und R₁₂ je Methyl oder Ethyl oder NR₁₁R₁₂ Pyrrolidinyl,
Y₅ Methyl oder Ethyl,
X₂ Wasserstoff, Methoxy oder Ethoxy und
Z₆ C₁-C₈-Alkyl bedeuten.

5. Phthalide gemäss Anspruch 3, **dadurch gekennzeichnet, dass** in Formel (3)
A₂ einen unsubstituierten oder durch Dimethylamino substituierten 1,2-Benzorest,
B₂ einen substituierten Phenylrest der Formel (3a),
Q₂ 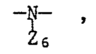 V₅ Wasserstoff oder Chlor,
V₆ Niederalkoxy,
R₁₁ und R₁₂ je Methyl, Ethyl oder -NR₁₁R₁₂ Pyrrolidinyl,
Y₅ Methyl oder Ethyl,
X₂ Wasserstoff, Methoxy oder Ethoxy und
Z₆ C₁-C₈-Alkyl bedeuten.

6. Verfahren zur Herstellung von Phthaliden gemäss Anspruch 1, **dadurch gekennzeichnet, dass** man in beliebiger Reihenfolge ein Mol eines Anhydrids der Formel 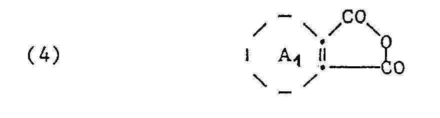 mit einem Mol einer Verbindung der Formel 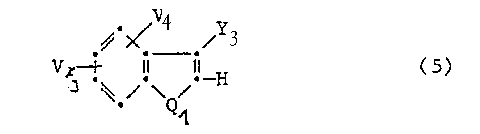 und einem Mol einer Verbindung der Formel
B₁-H (6)
umsetzt, worin in den Formeln (4), (5) und (6) A₁,B₁,Q₁,V₃, V₄, und Y₃ die in Anspruch 1 angegebene Bedeutung haben.

7. Verfahren gemäss Anspruch 6, **dadurch gekennzeichnet, dass** man eine Verbindung der Formel (5) mit einer Verbindung der Formel 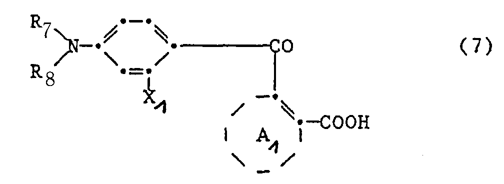 oder der Formel 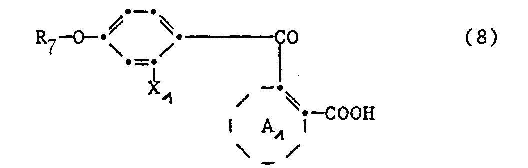 oder der Formel 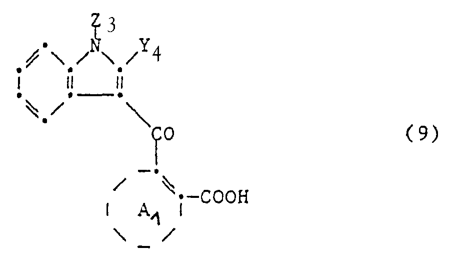 umsetzt, worin in den Formeln (7), (8) und (9) A₁, R₇, R₈, X₁, Z₃ und Y₄ die in Anspruch 1 angegebene Bedeutung haben.

8. Verwendung einer Phthalid verbindung der in einem der Ansprüche 1 bis 5 angegebenen Formel als Farbbildner in einem druckempfindlichen oder wärmeempfindlichen Aufzeichnungsmaterial.

9. Druck- oder wärmeempfindliches Aufzeichnungsmaterial, **dadurch gekennzeichnet, dass** es in seinem Farbreaktantensystem als Farbbildner mindestens eine Phthalid-verbindung der in einem der Ansprüche 1 bis 5 angegebenen Formel enthält.

10. Druckempfindliches Aufzeichnungsmaterial gemäss Anspruch 9, **dadurch gekennzeichnet, dass** es die Phthalidverbindung, gelöst in einem organischen Lösungsmittel, und mindestens einen festen Elektronenakzeptor enthält.

11. Druckempfindliches Aufzeichnungsmaterial gemäss einem der Ansprüche 9 oder 10, **dadurch gekennzeichnet, dass** die Phthalidverbindung in Mikrokapseln eingekapselt ist.

12. Druckempfindliches Aufzeichnungsmaterial gemäss Anspruch 11, **dadurch gekennzeichnet, dass** die eingekapselte Phthalidverbindung in Form einer Schicht auf der Rückseite eines Uebertragungsblattes und der Elektronenakzeptor in Form einer Schicht auf der Vorderseite des Empfangsblattes vorhanden sind.

13. Druckempfindliches Aufzeichnungsmaterial gemäss einem der Ansprüche 8 bis 11, **dadurch gekennzeichnet, dass** die Phthalidverbindung gemeinsam mit einem oder mehreren anderen Farbbildnern enthalten ist.

14. Wärmeempfindliches Aufzeichnungsmaterial gemäss Anspruch 8, **dadurch gekennzeichnet, dass** es in mindestens einer Schicht mindestens eine Phthalid verbindung der in einem der Ansprüche 1 bis 5 angegebene Formel, einen Elektronenakzeptor und gegebenenfalls ein Bindemittel und/oder Wachs enthält.

## Claims

1. A chromogenic phthalide of the formula 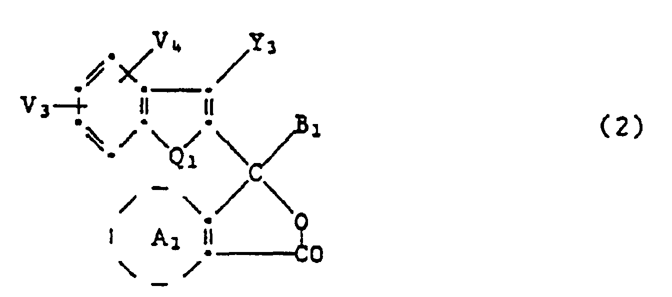 in which
V₃ is lower alkoxy or -NR₅R₆,
V₄ is hydrogen, halogen or lower alkyl,
A₁ is an unsubstituted or halogen- or di-lower alkylamino-substituted 1,2-benzo radical,
B is a substituted phenyl radical of the formula 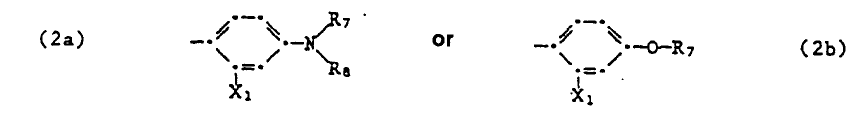 or a 3-indolyl radical of the formula 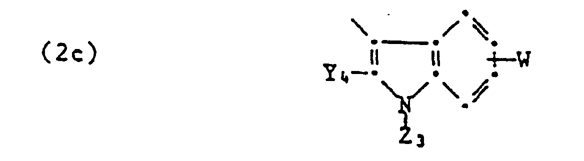 Q₁ is 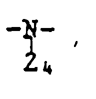 R₅, R₆, R₇ and R₈ are each, independently of the others, alkyl having at most 12 carbon atoms, C₅-C₆cycloalkyl, benzyl, phenethyl or phenyl, or the substituent pairs (R₅ and R₆) and (R₇ and R₈) are each, independently of the other, together with the nitrogen atom joining them, pyrrolidino, piperidino or morpholino,
Y₃ is lower alkyl,
Y₄ is hydrogen, methyl or phenyl,
Z₃ and Z₄ are each, independently of the other, hydrogen, C₁-C₈alkyl, acetyl, propionyl or benzyl,
X₁ is hydrogen, halogen, lower alkyl, C₁-C₈alkoxy, benzyloxy or the group -NT₃T₄,
T₃ and T₄ are each, independently of the other, hydrogen, lower alkyl, lower alkylcarbonyl or benzoyl, and
W is hydrogen or halogen.

2. A phthalide according to claim 1, wherein in formula (2) B₁ is a substituted phenyl radical of the formula (2a) or (2b).

3. A phthalide according to claim 1, of the formula 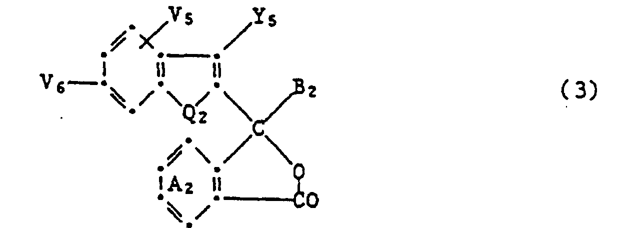 in which
the benzene ring A₂ is unsubstituted or substituted by lower alkylamino or halogen,
B₂ is a substituted phenyl radical of the formula 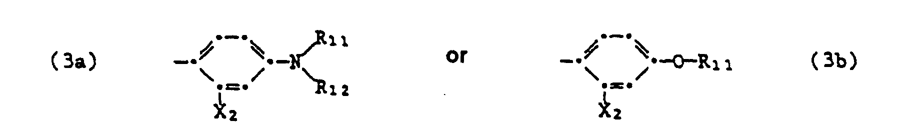 or a 3-indolyl radical of the formula 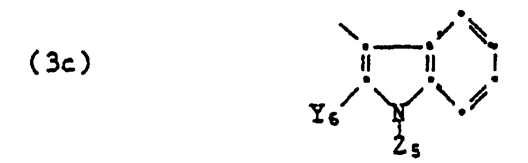 Q₂ is 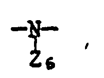 V₅ is hydrogen or halogen,
V₆ is -NR₉R₁₀ or lower alkoxy,
R₉, R₁₀, R₁₁ and R₁₂ are each, independently of the others, lower alkyl, cyclohexyl or benzyl, or the substituent groups -NR₉R₁₀ and NR₁₁R₁₂ are each, independently of the other, pyrrolidino, piperidino or morpholino,
X₂ is hydrogen, methyl, lower alkoxy, benzyloxy, acetylamino, propionylamino, benzoylamino or di-lower alkylamino,
Y₅ is lower alkyl,
Y₆ is methyl or phenyl, and
Z₅ and Z₆ are each, independently of the other, hydrogen, alkyl having 1 to 8 carbon atoms or benzyl.

4. A phthalide according to claim 3, wherein in formula (3)
A₂ is an unsubstituted or dimethylamino-substituted 1,2-benzo radical,
B₂ is a substituted phenyl radical of the formula (3a) or (3b),
Q₂ is 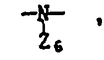 V₅ is hydrogen or chlorine,
V₆ is -NR₉R₁₀,
R₉ is methyl, ethyl or cyclohexyl,
R₁₀ is methyl or ethyl or -NR₉R₁₀ is pyrrolidinyl,
R₁₁ and R₁₂ are each methyl or ethyl or NR₁₁R₁₂ is pyrrolidinyl,
Y₅ is methyl or ethyl,
X₂ is hydrogen, methoxy or ethoxy, and
Z₆ is C₁-C₈alkyl.

5. A phthalide according to claim 3, wherein in formula (3)
A₂ is an unsubstituted or dimethylamino-substituted 1,2-benzo radical,
B₂ is a substituted phenyl radical of the formula (3a),
Q₂ is 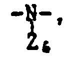 V₅ is hydrogen or chlorine,
V₆ is lower alkoxy,
R₁₁ and R₁₂ are each methyl or ethyl, or -NR₁₁R₁₂ is pyrrolidinyl,
Y₅ is methyl or ethyl,
X₂ is hydrogen, methoxy or ethoxy, and
Z₆ is C₁-C₈alkyl.

6. A process for preparing phthalides according to claim 1, which comprises reacting in any order one mole of an anhydride of the formula 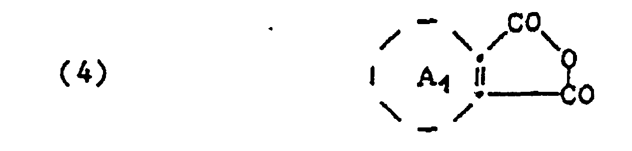 with one mole of a compound of the formula 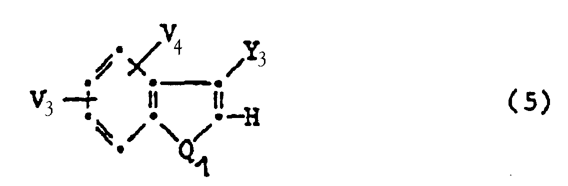 and one mole of a compound of the formula
B₁-H (6)
in which A₁, B₁, Q₁, V₃, V₄ and Y₃ in the formulae (4), (5) and (6) are as defined in claim 1.

7. A process according to claim 6, wherein a compound of the formula (5) is reacted with a compound of the formula 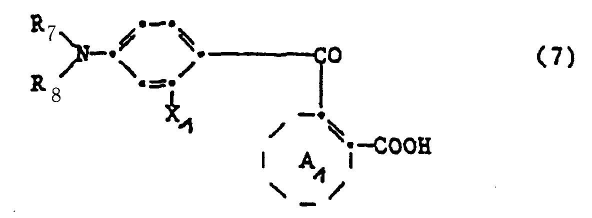 or of the formula 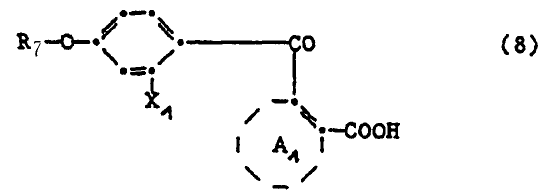 or of the formula 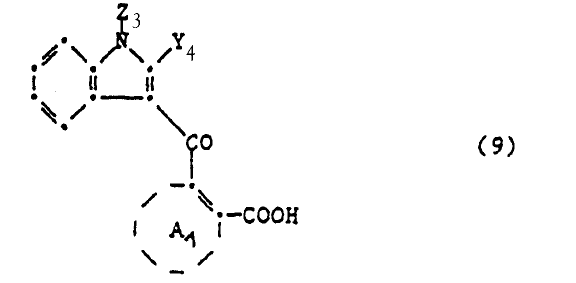 in which A₁, R₇, R₈, X₁, Z₃ and Y₄ in the formulae (7), (8) and (9) are as defined in claim 1.

8. The use of a phthalide compound of the formula given in any of claims 1 to 5 as a colour former in a pressure- or heat-sensitive recording material.

9. A pressure- or heat-sensitive recording material, comprising in its colour reactant system, as colour former, at least one phthalide compound of the formula given in any of claims 1 to 5.

10. A pressure-sensitive recording material according to claim 9, comprising the phthalide compound dissolved in an organic solvent, and at least one solid electron acceptor.

11. A pressure-sensitive recording material according to claim 9 or 10, wherein the phthalide compound is encapsulated in microcapsules.

12. A pressure-sensitive recording material according to claim 11, wherein the encapsulated phthalide compound is present in the form of a layer on the back of a transfer sheet and the electron acceptor is present in the form of a layer on the front of the reciever sheet.

13. A pressure-sensitive recording material according to one of claims 8 to 11, wherein the phthalide compound is present together with one or more other colour formers.

14. A heat-sensitive recording material according to claim 8, comprising in at least one layer at least one phthalide compound of the formula given in any of claims 1 to 5, an electron acceptor and, if desired, a binder and/or wax.

## Revendications

1. Phtalides chromogènes de formule 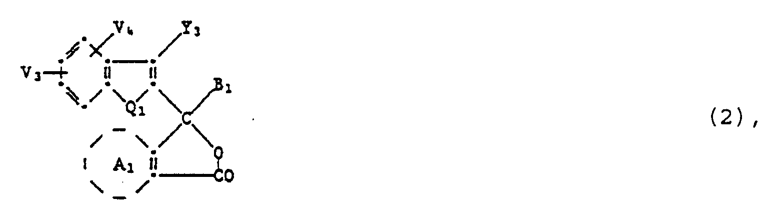 où V₃ représente un groupe alkoxy inférieur ou -NR₅R₆,
V₄ représente un atome d'hydrogène, un atome d'halogène ou un groupe alkyle inférieur,
A₁ représente un reste 1,2-benzène éventuellement substitué par un substituant halogène ou dialkylamino inférieur,
B₁ représente un reste phényle substitué de formule 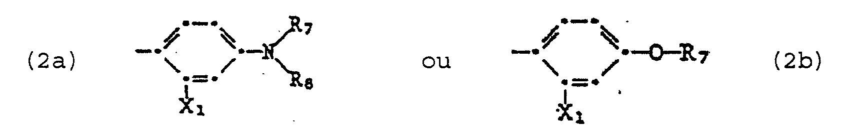 ou un reste 3-indolyle de formule 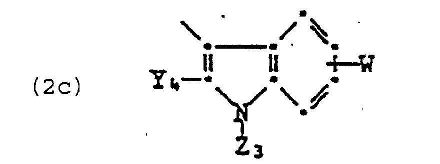 Q₁ représente un groupe 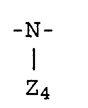 R₅, R₆, R₇ et R₈ représentent chacun, indépendamment les uns des autres, des groupes alkyle présentant au maximim 12 atomes de carbone ou cycloalkyle présentant 5 ou 6 atomes de carbone, benzyle, phénéthyle ou phényle, ou
les paires de substituants (R₅ et R₆) et (R₇ et R₈) représentent indépendamment, à chaque fois conjointement avec l'atome d'azote les reliant, un reste pyrrolidino, pipéridino ou morpholino
Y₃ représente un groupe alkyle inférieur,
Y₄ représente un atome d'hydrogène, un groupe phényle ou méthyle,
Z₃ et Z₄ représentent chacun, indépendamment l'un de l'autre, un atome d'hydrogène, des groupes alkyle en C₁-C₈, acétyle, propionyle ou benzyle,
X₁ représente un atome d'hydrogène, un atome d'halogène, des groupes alkyle inférieur, alkoxy en C₁-C₈, benzyloxy ou le groupe -NT₃T₄,
T₃ et T₄ représentent chacun, indépendamment l'un de l'autre, un atome d'hydrogène, des groupes alkyle inférieur, alkylcarbonyle inférieur ou benzoyle et
W représente un atome d'hydrogène ou un atome d'halogène.

2. Phtalides selon la revendication 1,
**caractérisés en ce que** dans la formule (2), B₁ représente un reste phényle substitué de formule (2a) ou (2b).

3. Phtalides selon la revendication 1,
**caractérisés en ce qu'**ils répondent à la formule 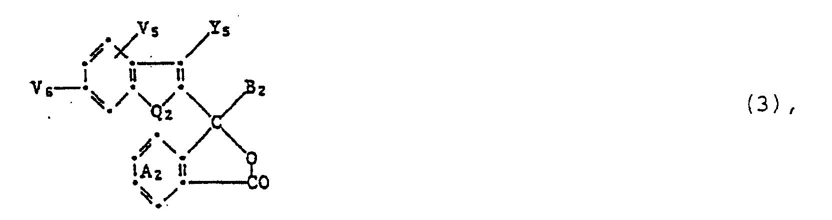 où le cycle benzénique A₂ est non substitué ou substitué par un substituant alkylamino inférieur ou halogène,
B₂ représente un reste phényle substitué de formule 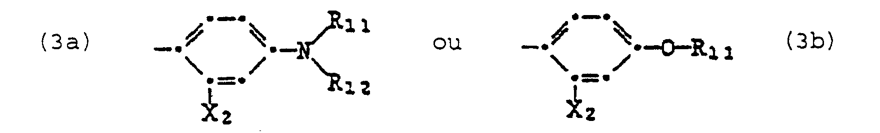 ou un reste 3-indolyle de formule 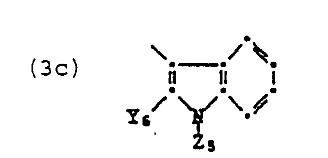 Q₂ représente un groupe 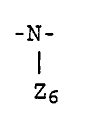 V₅ représente un atome d'hydrogène ou un atome d'halogène,
V₆ représente un groupe -NR₉R₁₀ ou alkoxy inférieur
R₉, R₁₀, R₁₁ et R₁₂ représentent chacun, indépendamment l'un de l'autre, des groupes alkyle inférieur, cyclohexyle ou benzyle ou les groupes substituants -NR₉R₁₀ et -NR₁₁R₁₂ pyrrolidino, pipéridino ou morpholino,
X₂ représente un atome d'hydrogène, des groupes méthyle, alkoxy inférieur, benzyloxy, acétylamino, propionyl-amino, benzoylamino ou dialkylamino inférieur,
Y₅ représente un groupe alkyle inférieur
Y₆ représente un groupe méthyle ou phényle, et
Z₅ et Z₆ représentent chacun, indépendamment l'un de l'autre, des atomes d'hydrogène, des groupes alkyle présentant 1 à 8 atomes de carbone ou benzyle.

4. Phtalides selon la revendication 3,
**caractérisés en ce que** dans la formule (3)
A₂ représente un reste 1,2-benzénique non substitué ou substitué par un substituant diméthylamino,
B₂ représente un reste phényle substitué de formule (3a) ou (3b),
Q₂ représente un groupe 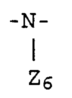 V₅ représente un atome d'hydrogène ou de chlore,
V₆ représente un groupe -NR₉R₁₀,
R₉ représente des groupes méthyle, éthyle ou cyclohexyle,
R₁₀ représente des groupes méthyle ou éthyle ou -NR₉R₁₀ pyrrolidinyle,
R₁₁ et R₁₂ représentent chacun des groupes méthyle ou éthyle ou -NR₁₁R₁₂ pyrrolidinyle,
Y₅ représente un groupe méthyle ou éthyle,
X₂ représente un atome d'hydrogène, des groupes méthoxy ou éthoxy et
Z₆ représente un groupe alkyle en C₁-C₈.

5. Phtalides selon la revendication 3,
**caractérisés en ce que** dans la formule (3)
A₂ représente un reste 1,2-benzénique non substitué ou substitué par un substituant diméthylamino,
B₂ représente un reste phényle substitué de formule (3a),
Q₂ représente un groupe 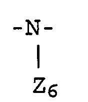 V₅ représente un atome d'hydrogène ou de chlore,
V₆ représente un groupe alkoxy inférieur,
R₁₁ et R₁₂ représentent chacun des groupes méthyle, éthyle ou -NR₁₁R₁₂ pyrrolidinyle,
Y₅ représente un groupe méthyle ou éthyle,
X₂ représente un atome d'hydrogène, des groupes méthoxy ou éthoxy et
Z₆ représente un groupe alkyle en C₁-C₈.

6. Procédé pour la préparation de phtalides selon la revendication 1, **caractérisé en ce qu'**on fait réagir dans un ordre arbitraire une mole d'un anhydride de formule 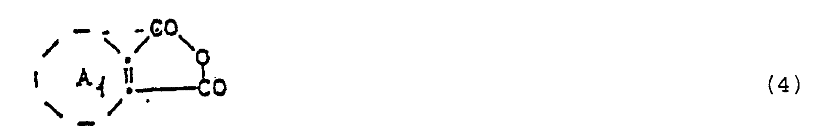 avec une mole d'un composé de formule 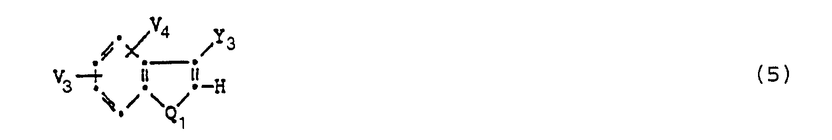 et une mole d'un composé de formule
B₁-H (6),
où dans les formules (4), (5) et (6) A₁, B₁, Q₁, V₃, V₄ et Y₃ possèdent la signification donnée.

7. Procédé selon la revendication 6,
**caractérisé en ce qu'**on fait réagir un composé de formule (5) avec un composé de formule 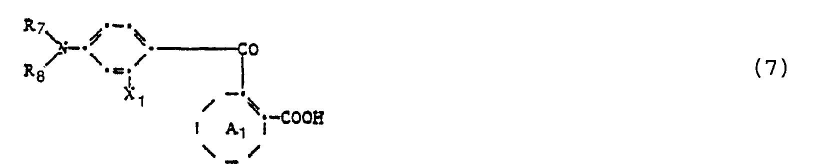 ou de formule 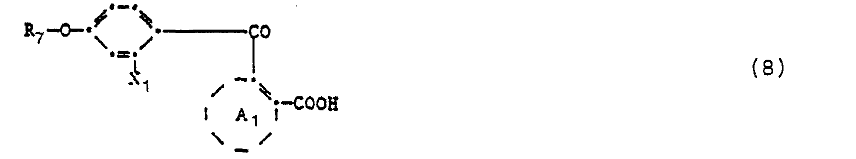 ou de formule 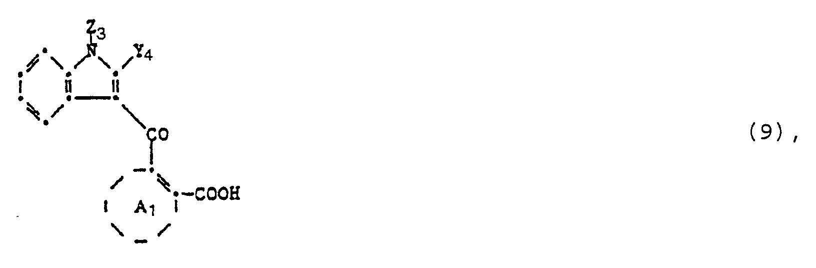 où dans les formules (7), (8) et (9) A₁, R₇, R₈, X₁, Z₃ et Y₄ possèdent la signification donnée.

8. Utilisation d'un composé du type phtalide de formule indiquée à l'une des revendications 1 à 5 en tant que coupleur coloré dans une matière d'enregistrement sensible à la pression ou sensible à la chaleur.

9. Matière d'enregistrement sensible à la pression ou sensible à la chaleur, **caractérisée en ce qu'**elle contient dans son système de réactants chromogènes en tant que coupleur coloré au moins un composé du type phtalide de formule indiquée à l'une des revendications 1 à 5.

10. Matière d'enregistrement sensible à la pression selon la revendication 9, **caractérisée en ce qu'**elle contient le composé du type phtalide dissous dans un solvant organique et au moins un accepteur d'électrons solide.

11. Matière d'enregistrement sensible à la pression selon l'une des revendications 9 ou 10, **caractérisée en ce que** le composé du type phtalide est encapsulé dans des microcapsules.

12. Matière d'enregistrement sensible à la pression selon la revendication 11, **caractérisée en ce que** le composé du type phtalide encapsulé est présent sous forme d'une couche sur le côté verso d'une feuille de transfert et l'accepteur d'électrons est présent sous forme d'une couche sur le côté recto de la feuille de réception.

13. Matière d'enregistrement sensible à la pression selon l'une des revendications 8 à 11, **caractérisée en ce que** le composé du type phtalide est contenu conjointement avec un ou plusieurs autres coupleurs colorés.

14. Matière d'enregistrement sensible à la pression selon la revendication 8, **caractérisée en ce que** au moins une couche contient au moins un composé du type phtalide de formule donnée à l'une des revendications 1 à 5, un accepteur d'électrons et éventuellement un liant et/ou cire.
